(19) Europäisches Patentamt
European Patent Office
Office européen des brevets

(11) Veröffentlichungsnummer: **0 012 344**
B1

# (12) EUROPÄISCHE PATENTSCHRIFT

(45) Veröffentlichungstag der Patentschrift: **12.08.81**

(51) Int. Cl.³: **C 07 F 9/65,** A 01 N 57/16 // C07D231/18

(21) Anmeldenummer: **79104928.1**

(22) Anmeldetag: **05.12.79**

(54) **O-Pyrazol(4)yl-O-äthyl-S-n-propyl-(thiono)thiol-phosphorsäureester, Verfahren zu ihrer Herstellung und ihre Verwendung als Schädlingsbekämpfungsmittel.**

(30) Priorität: **16.12.78 DE 2854389**

(43) Veröffentlichungstag der Anmeldung: **25.06.80 Patentblatt 80/13**

(45) Bekanntmachung des Hinweises auf die Patenterteilung: **12.08.81 Patentblatt 81/32**

(84) Benannte Vertragsstaaten: **AT BE CH DE FR GB IT NL SE**

(56) Entgegenhaltungen:
**DE-A-2 603 215**
**DE-A-2 639 258**

(73) Patentinhaber: **BAYER AG, Zentralbereich Patente, Marken und Lizenzen, D-5090 Leverkusen 1, Bayerwerk (DE)**

(72) Erfinder: **Maurer, Fritz, Dr., Roeberstrasse 8, D-5600 Wuppertal 1 (DE)**
Erfinder: **Hammann, Ingeborg, Dr., Belfortstrasse 9, D-5000 Köln (DE)**
Erfinder: **Homeyer, Bernhard, Dr., Obere Strasse 28, D-5090 Leverkusen 3 (DE)**

EP 0 012 344 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# O-Pyrazol(4)yl-O-äthyl-S-n-propyl-(thiono)thiol-phosphorsäureester, Verfahren zu ihrer Herstellung und ihre Verwendung als Schädlingsbekämpfungsmittel

Die Erfindung betrifft neue O-Pyrazol(4)yl-O-äthyl-S-n-propyl-(thiono)thiol-phosphorsäureester, ein Verfahren zu ihrer Herstellung sowie ihre Verwendung als Schädlingsbekämpfungsmittel, insbesondere als Insektizide, Akarizide und Nematizide.

Es ist bekannt, daß bestimmte O-Pyrazol(5)yl-O-äthyl-S-n-propyl-thionothiolphosphorsäureester, wie z. B. O-(4-Äthoxy-1-methyl-pyrazol(5)yl)- und O-(4-Methylthio-1-methyl-pyrazol(5)yl)-O-äthyl-S-n-propyl-(thiono)thiolphosphorsäureester, pestizid wirksam sind (vergleiche DE-OS 2 603 215).

Die Wirkung dieser Verbindungen ist jedoch, insbesondere bei niedrigen Wirkstoffkonzentrationen und Aufwandmengen, nicht immer ganz zufriedenstellend.

Es wurden nun neue O-Pyrazol(4)yl-O-äthyl-S-n-propyl-(thiono)thiol-phosphorsäureester der Formel

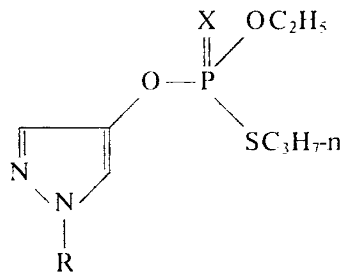
(I)

gefunden, in welcher

R für Alkyl, Cycloalkyl oder Phenyl steht und
X für Sauerstoff oder Schwefel steht.

Man erhält Verbindungen der Formel (I), wenn man 4-Hydroxypyrazole der Formel

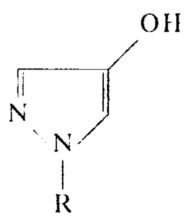
(II)

in welcher

R die oben angegebene Bedeutung hat,

mit O-Äthyl-S-n-propyl-(di)thiophosphorsäurediesterhalogeniden der Formel

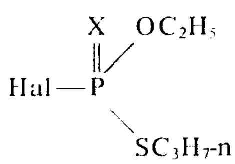
(III)

in welcher

X die oben angegebene Bedeutung hat und
Hal für Chlor oder Brom, insbesondere für Chlor, steht,

gegebenenfalls in Gegenwart eines Säureakzeptors und gegebenenfalls unter Verwendung eines Verdünnungsmittels umgesetzt.

Die neuen O-Pyrazol(4)yl-O-äthyl-S-n-propyl-(thiono)-thiolphosphorsäureester der Formel (I) zeichnen sich durch hohe Wirksamkeit als Schädlingsbekämpfungsmittel, insbesondere durch hohe insektizide, akarizide und nematizide Wirksamkeit aus.

Überraschenderweise zeigen die erfindungsgemäßen Verbindungen der Formel (I) erheblich höhere insektizide, akarizide und nematizide Wirkung als bekannte Verbindungen ähnlicher Konstitution und gleicher Wirkungsrichtung.

Die Erfindung betrifft insbesondere Verbindungen der Formel (I), in welcher

R für geradkettiges oder verzweigtes Alkyl mit 1 bis 5 Kohlenstoffatomen, Cycloalkyl mit 3 bis 8 Kohlenstoffatomen oder Phenyl steht und

X für Sauerstoff oder Schwefel steht.

Verwendet man beispielsweise 1-Methyl-4-hydroxy-pyrazol und O-Äthyl-S-n-propyl-thiolphosphorsäurediesterchlorid als Ausgangsverbindungen, so kann die Reaktion dieser Verbindungen durch folgendes Formelschema skizziert werden:

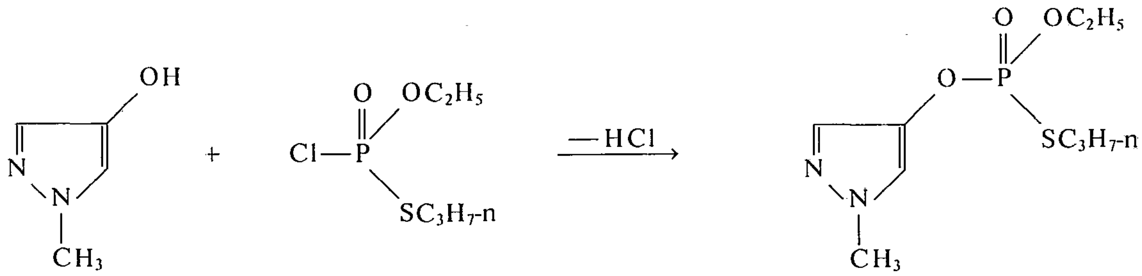


Die als Ausgangsstoffe zu verwendenden 4-Hydroxy-pyrazole sind durch Formel (II) definiert. Vorzugsweise steht darin

R für geradkettiges oder verzweigtes Alkyl mit 1 bis 5 Kohlenstoffatomen, Cycloalkyl mit 3 bis 8 Kohlenstoffatomen oder Phenyl.

Als Beispiele seien genannt:

1-Methyl-, 1-Äthyl-, 1-n-Propyl-, 1-iso-Propyl-, 1-n-Butyl-, 1-iso-Butyl-, 1-sek.-Butyl-, 1-tert.-Butyl-, 1-n-Pentyl-, 1-iso-Pentyl-, 1-sek.-Pentyl-, 1-tert.-Pentyl-, 1-(1-Äthyl)-propyl-, 1-Cyclopropyl-, 1-Cyclobutyl-, 1-Cyclopentyl-, 1-Cyclohexyl-, 1-Cycloheptyl-, 1-Cyclooctyl- und 1-Phenyl-4-hydroxy-pyrazol.

Die 4-Hydroxy-pyrazole der Formel (II) sind teilweise bekannt (vergleiche Liebigs Ann. Chem. 313 [1900], 17). Man erhält sie beispielsweise durch Umsetzung von bekannten 4-Methoxy-pyrazolen mit Bromwasserstoffsäure. Die Herstellung der 4-Methoxy-pyrazole erfolgt auf bekannte Weise aus Hydrazinen und 2-Methoxv-3-dimethyl-amino-acrolein (vergleiche Archiv der Pharmazie 300 (1967), 704–708).

Als Beispiele für die als Ausgangsstoffe zu verwendenden O-Äthyl-S-n-propyl-(di)thiophosphorsäurediesterhalogenide (III) seien genannt:

O-Äthyl-S-n-propyl-thiolphosphorsäurediesterchlorid und
O-Äthyl-S-n-propyl-dithiophosphorsäurediesterchlorid.

Diese Verbindungen sind bereits bekannt.

Das Verfahren zur Herstellung der erfindungsgemäßen O-Pyrazol(4)yl-O-äthyl-S-n-propyl(thiono)-thiolphosphorsäureester wird bevorzugt unter Verwendung geeigneter Lösungs- oder Verdünnungsmittel durchgeführt. Als solche kommen praktisch alle inerten organischen Solventien infrage. Hierzu gehören insbesondere aliphatische und aromatische, gegebenenfalls chlorierte Kohlenwasserstoffe, wie Benzin, Benzol, Toluol, Xylol, Methylenchlorid, Chloroform, Tetrachlorkohlenstoff, Chlorbenzol und o-Dichlorbenzol, Äther wie Diäthyl- und Dibutyläther, Tetrahydrofuran und Dioxan, Ketone wie Aceton, Methyläthyl-, Methylisopropyl- und Methylisobutylketon sowie Nitrile wie Acetonitril und Propionitril.

Als Säureakzeptoren können alle üblichen Säurebindemittel Verwendung finden. Besonders bewährt haben sich Alkalicarbonate und -alkoholate, wie Natrium- und Kaliumcarbonat, Natrium- und Kaliummethylat bzw. -äthylat, ferner aliphatische, aromatische oder heterocyclische Amine, beispielsweise Triäthylamin, Trimethylamin, Dimethylanilin, Dimethylbenzylamin und Pyridin.

Die Reaktionstemperatur kann innerhalb eines größeren Bereichs variiert werden. Im allgemeinen arbeitet man zwischen 0 und 100°C, vorzugsweise bei 10 bis 80°C. Das erfindungsgemäße Verfahren wird im allgemeinen bei Normaldruck durchgeführt.

Zur Durchführung des erfindungsgemäßen Verfahrens werden die Ausgangsstoffe gewöhnlich in äquimolaren Mengen eingesetzt. Ein Überschuß der einen oder anderen Reaktionskomponente bringt keine wesentlichen Vorteile. Die Umsetzung wird im allgemeinen in einem geeigneten Verdünnungsmittel in Gegenwart eines Säureakzeptors durchgeführt und das Reaktionsgemisch wird mehrere Stunden bei de- erforderlichen Temperatur gerührt. Danach gibt man ein organisches Lösungsmittel, z. B. Toluol, zu und arbeitet die organische Phase wie üblich durch Waschen, Trocknen und Abdestillieren des Lösungsmittels auf.

Die neuen Verbindungen fallen in Form von Ölen an, die sich zum Teil nicht unzersetzt destillieren lassen, jedoch durch sogenanntes »Andestillieren«, d. h. durch längeres Erhitzen unter vermindertem Druck, auf mäßig erhöhte Temperaturen von den letzten flüchtigen Anteilen befreit und auf diese Weise gereinigt werden. Zu ihrer Charakterisierung dient der Brechungsindex.

Die erfindungsgemäßen O-Pyrazol(4)yl-O-äthyl-S-n-propyl(thiono)thiol-phosphorsäureester zeichnen sich, wie bereits erwähnt, durch hervorragende insektizide, akarizide und nematizide Wirksamkeit aus.

Sie wirken gegen Pflanzenschädlinge wie auch gegen Hygiene- und Vorratsschädlinge und zeigen nur geringe Phytotoxizität.

Daher können die erfindungsgemäßen Verbindungen mit Erfolg im Pflanzenschutz sowie im Hygienebereich und Vorratsschutz als Schädlingsbekämpfungsmittel eingesetzt werden.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden die in der Landwirtschaft, in Forsten, im Vorrats- und Materialschutz sowie auf dem Hyginesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z. B. Oniscus asellus, Armadillidium vulgare, Porcellio scaber.

Aus der Ordnung der Diplopoda z. B. Blaniulus guttulatus.

Aus der Ordnung der Chilopoda z. B. Geophilus carpophaus, Scutigera spec.

Aus der Ordnung der Symphyla z. B. Scutigerella immaculata.

Aus der Ordnung der Thysanura z. B. Lepisma saccharina.

Aus der Ordnung der Collembola z. B. Onychiurus armatus.

Aus der Ordnung der Orthoptera z. B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.

Aus der Ordnung der Dermaptera z. B. Forficula auricularia.

Aus der Ordnung der Isoptera z. B. Reticulitermes spp.

Aus der Ordnung der Anoplura z. B. Phylloxera vastatrix, Pemphigus spp., Pediculus humanus corporis, Haematopinus spp., Linognathus spp.

Aus der Ordnung der Mallophaga z. B. Trichodectes spp., Damalinea spp.

Aus der Ordnung der Thysanoptera z. B. Hercinothrips fermoralis, Thrips tabaci.

Aus der Ordnung der Heteroptera z. B. Eurygaster spp., Dysdercus intermedius, Piesma quadrata, Cimex lectularius, Rhodnius prolixus, Triatoma spp.

Aus der Ordnung der Homoptera z. B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp., Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z. B. Pectinophora gossypiella, Bupalus piniarius, Cheimatobia brumata, Lithocolletis blancardella, Hyponomeuta padella, Plutella maculipennis, Malocosoma neustria, Euproctis chrysorrhoea, Lymantria spp. Bucculatrix thurberiella, Phyllocnistis citrella, Agrotis spp., Euxoa spp., Feltia spp. Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.

Aus der Ordnung der Coleoptera z. B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp., Trogoderma spp., Anthrenus spp., Attagenus spp., Lyctus spp., Meligethes aeneus, Ptinus spp., Niptus hololeucus, Gabbium psylloides, Tribolium spp., Tenebrio molitor, Agriotes spp., Conoderus spp., Melolontha melolontha, Amphimallon solstitialis, Costelytra zealandica.

Aus der Ordnung der Hymenoptera z. B. Diprion spp., Hoplocampa spp., Lasius spp., Monomorium pharaonis, Vespa spp.

Aus der Ordnung der Diptera z. B. Aedes spp., Anopheles spp., Culex spp., Drosophila melanogaster, Musca spp., Pannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinalla frit, Phorbia spp., Pegomyia hyoscyami, Ceratitis capitata, Dacus oleae, Tipula paludosa.

Aus der Ordnung der Siphonaptera z. B. Xenopsylla cheopis, Ceratophyllus spp.

Aus der Ordnung der Arachnida z. B. Scorpio maurus, Latrodectus mactans.
Aus der Ordnung der Acarina z. B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp.

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp.

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u. ä., sowie ULV-Kalt- und Warmnebel-Formulierungen.

Diese Formulierungen werden in bekannter Weise hergestellt, z. B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z. B. Erdölfraktionen, Alkohole, wie Butanol oder Glycol sowie deren Äther und Ester, Ketone, wie Aceton, Methyläthylketon, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z. B. Aerosol-Treibgas, wie Halogenkohlenwasserstoffe sowie Butan, Propan, Stickstoff und Kohlendioxid; als feste Trägerstoffe kommen in Frage: z. B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z. B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z. B. nichtionogene und anionische Emulgatoren, wie Polyoxyäthylen-Fettsäure-Ester, Polyoxyäthylen-Fettalkohol-Äther, z. B. Alkylarylpolyglykol-äther, Alkylsulfonate, Alkylsulfate, Arylsulfonate sowie Eiweißhydrolysate; als Dispergiermittel kommen in Frage: z. B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z. B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die Anwendung der erfindungsgemäßen Wirkstoffe erfolgt in Form ihrer handelsüblichen Formulierungen und/oder den aus diesen Formulierungen bereiteten Anwendungsformen.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,01 und 10 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Beispiel A
Plutella-Test

Lösungsmittel: 3 Gewichtsteile Aceton
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt

das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt.

Bei diesem Test zeigen z. B. die folgenden Verbindungen der Herstellungsbeispiele überlegene Wirksamkeit gegenüber dem Stand der Technik (Verbindung aus Beispiel 3 der DE-A 2 603 215): 2, 3, 4 und 12.

Beispiel B
Myzus-Test

Lösungsmittel: 3 Gewichtsteile Aceton
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea), die stark von der Pfirsichblattlaus (Myzus persicae) befallen sind, werden durch Tauchen in die Wirkstoffzubereitung der gewünschten Konzentration behandelt.

Nach der gewünschten Zeit wird die Abtötung in % bestimmt.

Bei diesem Test zeigen z. B. die folgenden Verbindungen der Herstellungsbeispiele überlegene Wirksamkeit gegenüber dem Stand der Technik (Verbindung aus Beispiel 3 der DE-A 2 603 215) 1, 4, 7 und 12.

Beispiel C
Tetranychus-Test (resistent)

Lösungsmittel: 3 Gewichtsteile Aceton
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris), die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt.

Bei diesem Test zeigen z. B. die folgenden Verbindungen der Herstellungsbeispiele überlegene Wirksamkeit gegenüber dem Stand der Technik (Verbindung aus Beispiel 2 der DE-A-2 603 215): 2, 3, 4, 7 und 12.

Beispiel D
Grenzkonzentrations-Test

Testnematode: Meloidogyne incognita
Lösungsmittel: 3 Gewichtsteile Aceton
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt, der mit den Testnematoden stark verseucht ist. Dabei spielt die Konzentration des Wirkstoffs in der Zubereitung praktisch keine Rolle, entscheidend ist allein die Wirkstoffmenge pro Volumeneinheit Boden, welche in ppm angegeben wird. Man füllt den behandelten Boden in Töpfe, sät Salat ein und hält die Töpfe bei einer Gewächshaus-Temperatur von 27° C.

Nach vier Wochen werden die Salatwurzeln auf Nematodenbefall (Wurzelgallen) untersucht und der Wirkungsgrad des Wirkstoffs in % bestimmt.

Bei diesem Test zeigen z. B. die folgenden Verbindungen der Herstellungsbeispiele überlegene Wirksamkeit gegenüber dem Stand der Technik (Verbindung aus Beispiel 2 der DE-A-2 603 215): 1, 2, 3 und 4.

## Herstellungsbeispiele

### Beispiel 1

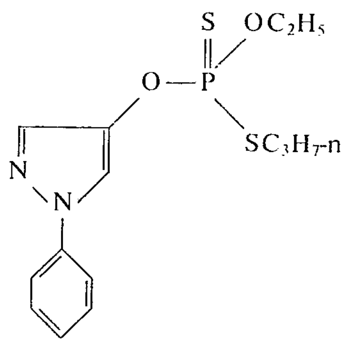

Ein Gemisch aus 16 g (0,1 Mol) 1-Phenyl-4-hydroxy-pyrazol, (Darstellung s. Wolff u. Fertig, Liebigs Ann. Chem. 313, 17 [1900]), 250 ml Acetonitril, 15,2 g (0,11 Mol) Kaliumcarbonat und 21,8 g (0,1 Mol) O-Äthyl-S-n-propyl-thionothiol-phosphorsäurediesterchlorid wird 6 Stunden bei 45°C gerührt. Dann gibt man 400 ml Toluol zu und schüttelt das Gemisch zwei mal mit je 300 ml Wasser. Die organische Phase wird über Natriumsulfat getrocknet und im Vakuum eingedampft. Der Rückstand wird bei 80°C andestilliert. Man erhält so 27,8 g (81% der Theorie) O-Äthyl-S-n-propyl-O-(l-phenyl-pyrazol(4)yl)-thiono-thiol-phosphorsäureester in Form eines gelben Öles mit dem Brechungsindex $n_D^{22}$ : 1,5816.

Analog Beispiel 1 können die folgenden Verbindungen der Formel

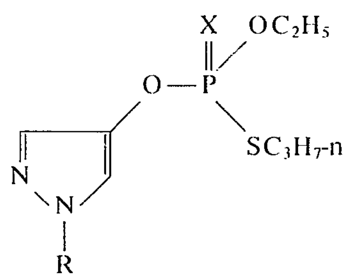

(I)

hergestellt werden:

| Beispiel Nr. | R | X | Ausbeute (% der Theorie) | Brechungsindex: |
|---|---|---|---|---|
| 2 | $C_3H_7$-iso | S | 78 | $n_D^{20}$ : 1,5242 |
| 3 | $C_3H_7$-iso | O | 39 | $n_D^{20}$ : 1,4948 |
| 4 | $C_2H_5$ | S | 75 | $n_D^{20}$ : 1,5334 |
| 5 | 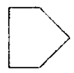 | S | 78 | $n_D^{20}$ : 1,5391 |
| 6 | 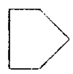 | O | | |
| 7 | $CH_3$ | S | 72 | $n_D^{20}$ : 1,5386 |
| 8 | $CH_3$ | O | | |
| 9 | $C_3H_7$-n | S | | |
| 10 | $C_3H_7$-n | O | | |
| 11 | $C_4H_9$-n | S | | |
| 12 | $C_4H_9$-sek. | S | 73 | $n_D^{20}$ : 1,5213 |
| 13 | $C_4H_9$-iso | S | | |
| 14 | $C_4H_9$-tert. | S | | |
| 15 | $CH(C_2H_5)_2$ | S | | |
| 16 | 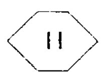 | S | | |

Die als Ausgangsmaterialien einzusetzenden 4-Hydroxypyrazole sind zum Teil bekannt. Sie können z. B. nach folgendem Verfahren hergestellt werden:

### Beispiel a

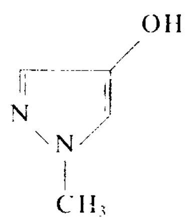

Eine Lösung von 11,2 g (0,1 Mol) 1-Methyl-4-methoxy-pyrazol (Darstellung s. H. Plümpe und E. Schegk, Archiv der Pharmazie 300, 704–708 (1967) in 70 ml 48%iger Bromwasserstoffsäure wird 18 Stunden unter Rückfluß gekocht und dann im Vakuum zur Trockne eingedampft. Der Rückstand wird in 50 ml Wasser gelöst, die Lösung durch Zugabe von Natriumhydrogencarbonat neutralisiert und dann 6 mal mit je 50 ml Chloroform extrahiert. Die organischen Phasen werden über Natriumsulfat getrocknet und im Vakuum eingedampft. Zurück bleiben 4,1 g (42% der Theorie) 1-Methyl-4-hydroxy-pyrazol in Form schwach gelber Kristalle mit dem Schmelzpunkt 71°C.

In analoger Weise können die folgenden Verbindungen der Formel

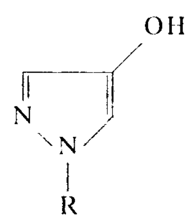

hergestellt werden:

| Beispiel Nr. | R | Ausbeute (% der Theorie) | Physikal. Daten (Schmelzpunkt C, Brechungsindex) |
|---|---|---|---|
| b | $C_3H_7$-iso | 70 | 63 |
| c | $C_2H_5$ | 65 | $n_D^{20}$ : 1,5078 |
| d | 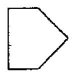 | 62 | 79 |
| e | $C_3H_7$-n | | |
| f | $C_4H_9$-n | | |
| g | $C_4H_9$-sek | 51 | $n_D^{20}$ : 1,4991 |
| h | $C_4H_9$-iso | | |
| i | $C_4H_9$-tert. | | |
| j | $CH(C_2H_5)_2$ | | |
| k | 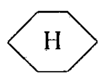 | | |

**Patentansprüche**

1. Verbindungen der Formel

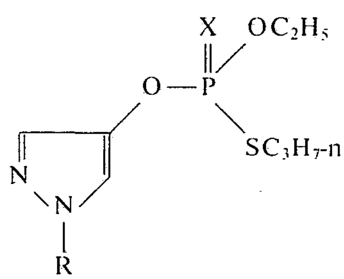

(I)

worin

R für Alkyl, Cycloalkyl oder Phenyl steht und
X für Sauerstoff oder Schwefel steht.

2. Verfahren zur Herstellung von Verbindungen der Formel

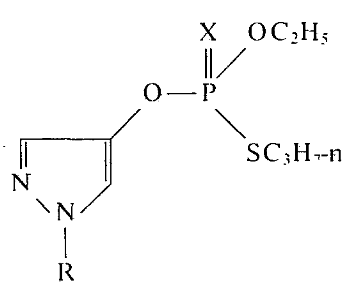

(I)

worin

R für Alkyl, Cycloalkyl oder Phenyl steht und
X für Sauerstoff oder Schwefel steht,

dadurch gekennzeichnet, daß man 4-Hydroxy-pyrazole der Formel

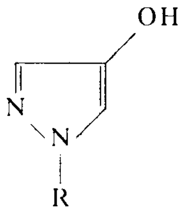

(II)

in welcher

R die oben angegebene Bedeutung hat,

mit O-Äthyl-S-n-propyl-(di)thiophosphorsäurediesterhalogeniden der Formel

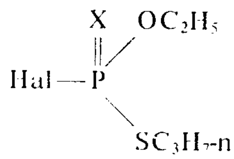

(III)

in welcher

X die oben angegebene Bedeutung hat und
Hal für Chlor oder Brom, insbesondere Chlor, steht,

gegebenenfalls in Gegenwart eines Säureakzeptors und gegebenenfalls unter Verwendung eines Verdünnungsmittels umsetzt.

3. Schädlingsbekämpfungsmittel, gekennzeichnet durch einen Gehalt an mindestens einer Verbindung gemäß Formel I.

4. Insektizides, nematizides und akarizides Mittel nach Anspruch 3, gekennzeichnet durch einen Gehalt an mindestens einer Verbindung gemäß Formel I.

5. Verwendung von Verbindungen gemäß Formel I zur Bekämpfung von Schädlingen, insbesondere von Insekten, Nematoden und Spinnentieren.

6. Verfahren zur Herstellung von Schädlingsbekämpfungsmitteln, insbesondere von insektiziden, akariziden und nematiziden Mitteln, dadurch gekennzeichnet, daß man Verbindungen der Formel I mit Streckmitteln und/oder oberflächenaktiven Mitteln vermischt.

**Claims**

1. Compounds of the formula

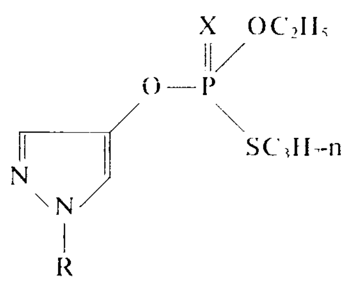

(I)

wherein

R represents alkyl, cycloalkyl or phenyl and
X represents oxygen or sulphur.

2. Process for the preparation of compounds of the formula

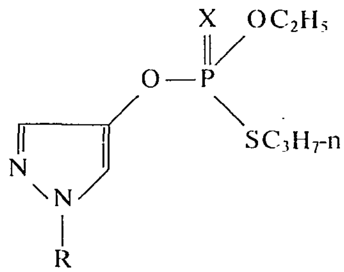

(I)

wherein

R represents alkyl, cycloalkyl or phenyl and
X represents oxygen or sulphur,

characterised in that 4-hydroxy-pyrazoles of the formula

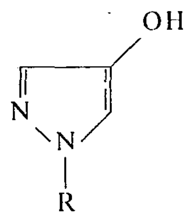

(II)

in which

R has the meaning indicated above,

are reacted with O-ethyl-S-n-propyl-(di)thiophosphoric acid diester halides of the formula

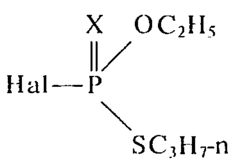

(III)

in which

X has the meaning indicated above and
Hal represents chlorine or bromine, in particular chlorine,

if appropriate in the presence of an acid acceptor and if appropriate using a diluent.

3. Agents for combating pests, characterised in that they contain at least one compound according to formula I.

4. Insecticidal, nematicidal and acaricidal agent according to Claim 3, characterised in that it contains at least one compound according to formula I.

5. Use of compounds according to formula I for combating pests, in particular insects, nematodes and arachnidae.

6. Process for the preparation of agents for combating pests, in particular insecticidal, acaricidal and nematicidal agents, characterised in that compounds of formula I are mixed with extenders and/or surface-active agents.

**Revendications**

1. Composés de formule

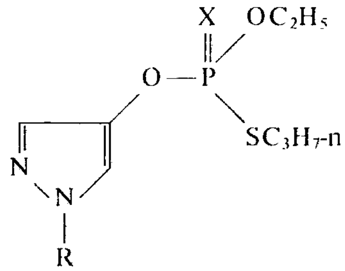

(I)

dans laquelle

R représente un groupe alkyle, cycloalkyle ou phényle et
X représente l'oxygène ou le soufre.

2. Procédé de préparation des composés de formule

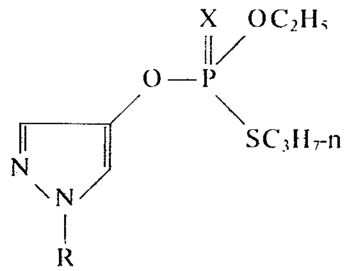

(I)

dans laquelle

R représente un groupe alkyle, cycloalkyle ou phényle et
X représente l'oxygène ou le soufre,

caractérisé en ce que l'on fait réagir des 4-hydroxy-pyrazoles de formule

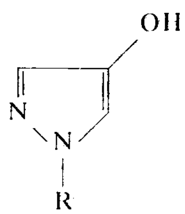

(II)

dans laquelle

R a les significations indiquées ci-dessus,

avec des halogénures de (di)thiophosphates d'O-éthyle et de S-n-propyle de formule

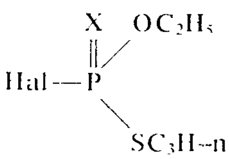

(III)

dans laquelle

X a les significations indiquées ci-dessus et
Hal représente le chlore ou le brome, plus spécialement le chlore,

éventuellement en présence d'un accepteur d'acide, et éventuellement avec utilisation d'un diluant.

3. Produit pesticide caractérisé en ce qu'il contient au moins un composé de formule I.

4. Produit insecticide, nématocide et acaricide selon la revendication 3, caractérisé en ce qu'il contient au moins un composé de formule I.

5. Utilisation des composés de formule I pour la lutte contre les parasites, en particulier les insectes, les nématodes et les acariens.

6. Procédé de préparation de produits pesticides, en particulier de produits insecticides, acaricides et nématocides, caractérisé en ce que l'on mélange des composés de formule I avec des diluants et/ou des agents tensio-actifs.